**Europäisches Patentamt**

**European Patent Office**

**Office européen des brevets**

(19)

(11) Publication number: **0 135 920**
**B1**

(12)     EUROPEAN PATENT SPECIFICATION

(45) Date of publication of patent specification: 26.08.87

(51) Int. Cl.⁴: **C 07 C 143/34, C 07 C 139/00**

(21) Application number: **84111243.6**

(22) Date of filing: **20.09.84**

(54) **Improved process for making alkaline-earth metal salts of alkaryl sulfonic acids.**

(30) Priority: **22.09.83 US 534905**

(43) Date of publication of application:
**03.04.85 Bulletin 85/14**

(45) Publication of the grant of the patent:
**26.08.87 Bulletin 87/35**

(84) Designated Contracting States:
**GB IT NL SE**

(56) References cited:
**FR-A-1 265 309**
**US-A-3 719 596**

(73) Proprietor: **DRESSER INDUSTRIES,INC.**
**The Dresser Building Elm & Akard Street**
**P.O. Box 718**
**Dallas Texas 75221 (US)**

(72) Inventor: **Stamatakis, Emanuel**
**3418 South Briar Knoll**
**Houston Texas (US)**
Inventor: **Sample, Thomas Earl**
**8015 Windswept**
**Houston Texas (US)**
Inventor: **Javora, Paul Henry**
**10366 Briar Forest**
**Houston Texas (US)**

(74) Representative: **Wagner, Karl H.**
**WAGNER & GEYER Patentanwälte**
**Gewuerzmuehlstrasse 5 Postfach 246**
**D-8000 München 22 (DE)**

Courier Press, Leamington Spa, England.

## Description

### Background of the invention

Alkaline-earth metal salts of alkylbenzene sulfonic acids find many commercial uses, including use as additives in lubricating oils; as corrosion inhibitors, in anti-freeze formulations for example; in rust-proofing compositions, in drilling mud formulations, and the like. As is obvious, these materials find many large volume uses and cost becomes an important factor. Manufacturing methods for preparing such materials are described in many patents. Generally alkylbenzene sulfonic acids are reacted with calcium hydroxide in an aqueous-organic solvent, and thereafter the water and most of the solvents are removed by distillation. Typical are US—A—3,719,596 and 2,799,784.

In US—A—3,719,596 there is described a method of preparing alkaline-earth alkylbenzene sulfonates. The process comprises forming a suspension of an alkaline-earth metal hydroxide with a water-immiscible azeotrope-forming liquid such as toluene, xylene, tetrachloroethylene or the like, and water, adding thereto a higher alkylbenzene sulfonic acid in a sufficient amount to form the double salt, lowering the pH to an acidic range thereby neutralizing the hydroxide, adding a lower alkanolamine having from 1 to 3 hydroxy groups such as diethylanolamine to neutralize the system and solubilize the salt, and finally, removing practically all of the water by distillation in the presence of a polyhydric alcohol such as ethylene glycol. Monohydric alcohols such as isopropanol are taught to be inappropriate for this use.

There is disclosed in US—A—2,799,784 a method for making alkaline earth metal sulfonates by heating a mixture of an oil-soluble alkaryl sulfonic acid and an alkaline earth metal compound, preferably in the presence of a solvent, with water at a temperature above 104°C (220°F) to 199°C (390°F) and under superatmospheric pressure, and removing the water by azeotropic distillation. The alkaline earth metal compound to neutralize the sulfonic acid includes alcholoates, borates, carbonates, carboxylate, hydroxides, hydrates, hydrosulfide, oxides, nitrate, sulfide, thiocarbonates, and others of magnesium, calcium, strontium and barium. The water used is present in amount equal to 5 (50%) to 100 parts per 10 parts of alkaline earth metal compound. The patent teaches that "When an organic sulfonic acid and an alkaline earth metal compound in excess of that required to neutralize the sulfonic acid are agitated together in the presence of an appreciable amount of water at a temperature above 104°C (220°F), and under sufficient pressure to hold the water in the liquid state, the water provides a condition whereby the excess alkaline earth metal compound forms a stable dispersion. If the reaction is carried out in an open kettle, the water escapes before the reactants reach the temperature at which dispersion of the alkaline earth metal compound is most suitably obtained."

In US—A—4,279,837, the alkyl benzene sulfonic acids are neutralized with the necessary amount of basic alkaline earth metal compound in an inert organic solvent in the presence of 1 to 10% by weight of a oxalkylate, and the mixture is then azeotropically distilled to remove the water and a large part of the organic solvent.

A less complex and less costly process for making solutions of alkaline earth metal salts of alkaryl sulfonic acids having a low water content is desired. The elimination of a costly azeotropic distillation step is particularly desirable since this would make possible the elimination of costly equipment, some reagents, waste storage, and disposal, would use less energy, and would result in more efficient use of existing facilities.

### Summary of the invention

A novel process for making solutions of alkaline-earth metal salts of alkaryl sulfonic acids that contain about 1.5 to less than 3.5 weight percent water and that does not require an azeotropic distillation step to obtain a product containing water at this level, comprises reacting an alkaryl sulfonic acid with alkaline-earth metal oxide and greater than 0.05 to less than about 2.5 weight percent water in an organic solvent.

### Detailed description

A number of advantages are realized from the novel process of this invention. Eliminating the azeotropic distillation step, that is an essential part of commercial processes also eliminates the need for expenditure of costly energy required for a distillation process, along with the equipment required for azeotropic distillation, and the cleaning and maintenance associated with the operation thereof. Also eliminated is the water containing distillate of the distillation step. The water had to be removed from this inert solvent before it could be used again, or if it was discarded, this represents a cost that is saved, also accompanied by the need for fewer storage vessels and other related equipment. Further, the total processing time per batch is reduced by eliminating this step, resulting in more production and lower cost per unit of equipment used. There are many other obvious advantages in the use of the novel process of this invention. The reaction does not need to be heated to reflux because it generates its own heat by reaction; nor is supratmospheric pressure required. While the defined solutions of this invention, containing less than 3 weight percent water, are obtained without requiring an azeotropic distillation step, should one wish to further reduce the water content of the solutions to a lower level, this can be economically done by utilizing the heat generated by the reaction in the process of this invention to distil off the desired amount of water. This represents an improvement over the prior art in that less water is present initially and it is not necessary to add heat to distil off the water, both being an economic advantage.

In the novel process of this invention, the necessary ingredients are a polar organic solvent, an alkaline-earth metal oxide, the defined amounts of water and an alkaryl sulfonic acid.

While any polar organic solvent is contemplated, normally used are alkanols containing 1 to 8 carbon atoms, such as ethanol, propanol, isopropanol, butanol, isobutanol, pentanol, heptanol, octanol, diols, such as ethylene glycol alone or with ethers such as butyl cellosolve, ethylene glycol alone or with ethers such as butyl cellosolve, ethylene glycol, monobutylether and the like. Isopropanol and isobutanol are useful alcohols for commercial operations. While the alcohol can be used alone, it has been found that excellent results are obtained when the alcohol is mixed with a non-polar solvent, normally a hydrocarbon solvent such as hexane, heptane, mineral spirits, benzene, cyclohexane and the like. Diesel fuel oil ASTM #2 has been found to be satisfactory when mixed with alcohols, including isopropanol or ethylene glycol. The amounts of polar solvents including alcohols used in polar/non-polar solvent mixtures normally will be from about 15 weight percent of the solvent mixture to 100 weight percent alkanol. About one-third or more of alcohol to about one-half are normally used with the non-polar solvent. With diesel oil, a useful ratio is about 60% diesel oil and about 40% isopropanol. The water content of the solvents is preferably below about 0.1 weight percent.

The process of this invention may be used to make alkaline-earth metal salts of a great variety of alkaryl sulfonic acids. The alkyl groups may contain from 3 to 30 carbon atoms, and the aryl groups may be derived from benzene, naphthalene, anthracene, and phenanthrene. More preferably, the alkyl groups contain 8 to 20 carbon atoms. A particularly useful group of alkylbenzene and alkylnaphthalene sulfonic acids have alkyl groups that contain 10 to 14 carbon atoms, such as decylbenzene sulfonic acid, dodecylbenzene sulfonic acid, tetradecane sulfonic acid, dodecylnaphthyl sulfonic acid and the like.

While the process may be practiced with other alkaline-earth metal oxides as magnesium oxide, strontium oxide, barium oxide and the like, particularly good results are obtained with calcium oxide. A stoichiometric amount of calcium oxide is normally used in relation to the alkaryl sulfonic acid, and a slight excess insures good conversion of the sulfonic acid groups to the salt.

The alkaline-earth metal oxide should be substantially free of lumps and preferably is in a finely divided state. One form that is satisfactory is pulverized so that 98 to 99.5% that will pass a No. 325 (0,044 mm) mesh sieve. Normally the particle size will range from about 0,07 to 0,038 mm) (200 to 400 mesh).

The amount of water present in the reaction is critical and control thereof is essential to the practice of the invention. The amount of water used must be such that the final product of the reaction mixture contains less than about 3.5 weight percent, preferably less than about 2.5 weight percent water so that no distillation step is required to obtain a product with this water content. The total amount of water present during the course of the reaction preferably should not be greater than about 2 weight percent of the total reactants charged, and less than 25 weight percent, preferably less than 10 weight percent of the amount of alkaline-earth metal oxide used. Normally the amount of water used in the reaction will be greater than about 0.05 weight percent, preferably greater than about 0.10 weight percent to less than about 2 weight percent. A practical amount of water present in the reactor before the alkaryl sulfonic acid is added is about 0.15 to about 0.5 weight percent of the total reactants used, preferably about 0.20 to 0.25. The amount of water in the other reactants, if any, is such that the overall amount of water in the final product is less than about 3.5%. Practically the solvents and the alkaryl sulfonic acid should contain less than about 1 weight percent water, normally less than about 0.5 weight percent water, and normally are water free. One convenient method to add the water to the reaction mixture and as an aid in controlling the amount used, is to use as one of the materials in the reaction, at least about 5 to about 10 weight percent or more of a calcium alkaryl sulfonate solution, containing about 40 to 70 weight percent of calcium alkaryl sulfonate and less than about 2.5 weight percent water, added to the reaction mixture before addition of the alkaryl sulfonic acid. These amounts normally will be enough to provide the desired amount of water. The source may be from a commercial preparation of the alkaline-earth metal alkaryl sulfonate. Materials contemplated include solutions prepared by commercial processes. Such materials may contain as much as 4 weight percent or more water, and the amounts of solution used normally will be adjusted to meet the criteria for water present set forth above. Preferably these starter or seed materials contain less than 5 weight percent water, the remainder normally being the solvent used and the alkaryl sulfonate. The total solids of the solution normally vary from about 40 to about 70 weight percent, but may be less or more as desired such as about 20 to 80 weight percent. Solvent-free sulfonates containing the defined amounts of water may be used. Use of these materials provides a particularly useful way to add and control the amount of water required and used, and results in an excellent reaction, particularly in the initial stages. Addition of water by this method is unique in that the water required to initiate the reaction does not add to or increase the final weight percent of water based on the total solids in the final product.

Preferably the alkaryl sulfonic acid is proportioned into the reaction mixture at a rate to initiate and sustain the salt forming reaction step, as evidenced by a continued increase in the reaction temperature, usually in commercial equipment to a reflux state of the reaction mixture. While high temperatures and supratmospheric pressure are not required, the reaction can be conducted in closed reactors. The rate to be used is readily determined by those skilled in the art. If the rate of addition is too slow, the reaction slows down, and if the rate of addition is too fast, the reaction will slow down, the temperature decrease, and the reaction may stop.

The pH of the reaction mixture, even after the reaction is complete, may be less than 7. Because of potential corrosion problems in storage and later use, this is normally adjusted to about 8 or 9 by addition of a basic material. Amines, such as alkanolamines including diethanolamine, have been found to be useful materials.

The practice of the invention is demonstrated in the following Examples.

Example I

62.8 weight parts of isopropanol, 0.55 weight part of water and 18.30 weight parts of calcium oxide were added to and mixed in a reactor equipped with an agitator and reflux means. A mixture of 210.6 weight parts of dodecylbenzene sulfonic acid and 69.8 weight parts of isopropanol was added to the reactor over a four hour period. The temperature of the reaction mixture at the beginning of the reaction was 23.9°C, and at the end of the four hour addition of the dodecylbenzene sulfonic acid, the temperature was 49.8°C. The reaction was conducted at atmospheric pressure. Essentially complete conversion of the dodecylbenzene sulfonic acid to the calcium salt was obtained. 2.65 weight parts of diethanolamine was added to raise the pH to 8.0. The water content of the reaction product solution was 2.41 weight percent.

Example II

147.7 kg (326 pounds) of No. 2 diesel oil (ASTM) was charged to a 1134 l (300 gallon) reactor and the agitator turned on. 85,6 kg (189 pounds) of isopropanol was then charged to the reactor, followed by 51,6 kg (114 pounds) of a solution containing 30,98 kg (68.4 pounds) of calcium dodecyl benzene sulfonate, 1,03 kg (2.28 pounds) of water, and 19,6 kg (43.3 pounds) of isopropanol from a previous reaction. 33,5 kg (94 pounds) of calcium oxide was then stirred into the mixture. When this was well mixed, the addition of 361,5 kg (798 pounds) of dodecylbenzene sulfonic acid was begun at a rate of 3,17 kg (7 pounds) per minute. This addition was completed in 2 hours. The reaction was conducted at atmospheric pressure. The initial temperature before addition of the dodecylbenzene sulfonic acid was begun was 32°C (90°F) and at the end of the addition, the temperature was 88°C (190°F). The reaction was allowed to continue for an additional 2 hour period. At the end of this period 15,4 kg (34 pounds) of diethylanolamine was added to adjust the pH to 8.05. The total solids of the solution was 61.0% and the water content was 2.0%.

Example III

To a 11340 l (3000 gallon) reactor there was charged 1977,8 kg (4,366 pounds) of No. 2 diesel oil, 1161,0 kg (2,563 pounds) of isopropanol, 1148,3 kg (2,535 pounds) of a solution from a previous reaction containing 689,0 kg (1,521 pounds) of calcium dodecylbenzene sulfonate, 22,96 kg (50.7 pounds) of water and, 436,2 kg (963 pounds) of isopropanol, and 475,6 kg (1,050 pounds) of calcium oxide, with agitation, and at a temperature of about 35°C (95°F). The reactor was closed and cooling water turned on. 4,893 kg (10,801 pounds) of dodecylbenzene sulfonic acid was added to the reactor at a rate of 26,5 l (7 gallons) per minute for the first 20 minutes and then the rate was increased so that the remainder of the dodecylbenzene sulfonic acid, 4131 l (1,093 gallons), was added over a 2 hour period. The temperature 10 minutes after this addition was begun was 52°C (125°F) and the cooling water was turned off. At the end of the addition the temperature was 128°C (263°F) and the cooling water was turned back on to lower the temperature to 90°C (195°F). The reaction was allowed to continue for 1 hour and a sample taken that had a pH of 3.38. After an additional 40 minutes 136,3 kg (301 pounds) of diethanolamine was added to the reactor. The final pH was 8.15, the total solids of the solution was 62.1% and the percent free water was 2.24%. The conversion to calcium dodecyl benzene sulfonate was substantially complete.

Example IV

To a reactor equipped with agitating and reflux means, 31.4 weight parts of isopropanol, 31.4 weight parts of a solution containing 18.6 weight parts of calcium dodecylbenzene sulfonate in 12.2 weight parts of isopropanol, and 0.628 weight parts of water was added with stirring. A mixture of 210.6 weight parts of dodecylbenzene sulfonic acid and 100.48 weight parts of isopropanol was added to the reactor over a three hour and 18 minute period. The mixture of reactants, before addition of the dodecylbenzene sulfonic acid was begun, was at room temperature, and at the end of the addition, had risen to 49.3°C. 1.25 weight parts of diethanolamine was added to adjust the pH of the reaction product to 8.5. The conversion was essentially 100%, and the water content of the reaction product was 2.79%.

Alkaline-earth metal alkaryl sulfonate solution prepared in accordance with the improved process of this invention are useful as emulsifiers and have excellent detergent properties. These materials are useful and find applications as lubricating oil additives, particularly in low water content lube oils; in corrosion inhibiting compositions; in storage rust-proofing compositions; in oil field applications to control corrosion; in oil field drilling mud compositions, and the like, as is well known to those skilled in the art.

**Claims**

1. A process for making alkaline-earth metal alkaryl sulfonate solutions containing 15 to about 3.5 weight percent water comprising reacting together an alkaline-earth metal oxide with an alkaryl sulfonic acid in a medium containing a polar solvent in the presence of greater than 0.05 to less than about 2.5 weight percent water.

2. The process of Claim 1 wherein the alkaryl sulfonic acids have alkyl groups containing 3 to 30 carbon atoms, the polar solvent is an alkanol and the amount of water used is from about 0.1 to less than about 2 weight percent.

3. The process of Claim 2 wherein the alkaline-earth metal oxide is calcium oxide, the alkaryl sulfonic acid is a benzene or naphthalene derivative wherein the alkyl groups contain 10 to 20 carbon atoms, the alkanol is isopropanol or isobutanol, and the amount of water initially present during the reaction is from about 0.15 to 0.5 weight percent.

4. The process of Claim 3 wherein the sulfonic acid is an alkylbenzene sulfonic acid wherein the alkyl groups contain 10 to 14 carbon atoms, and the alkanol is isopropanol.

5. The process of Claim 4 wherein the alkaryl benzene sulfonic acid is dodecylbenzene sulfonic acid.

6. The process of Claim 2 wherein the alkaryl sulfonic acid is proportioned into a mixture of said solvent medium, alkaline-earth metal oxide and water, at a rate to at least sustain the reaction of alkaline-earth metal oxide and the sulfonic acid to form the salt thereof.

7. The process of Claim 6 wherein the alkaline-earth metal oxide is calcium oxide, the alkaryl sulfonic acid is a benzene or naphthalene derivative, wherein the alkyl groups contain 10 to 20 carbon atoms, the alkanol is isopropanol or isobutanol, and the amount of water present during the reaction is greater than 0.15 weight percent.

8. The process of Claim 7 wherein the sulfonic acid is an alkylbenzene sulfonic acid wherein the alkyl groups contain 10 to 14 carbon atoms, and the alcohol is isopropanol.

9. The process of Claim 8 wherein the alkyl benzene sulfonic acid is dodecylbenzene sulfonic acid.

10. The process of Claim 6 wherein the solvent medium comprises a non-polar and a polar organic solvent, the polar solvent being present in the solvent medium in amount of at least 15 weight percent.

11. The process of Claim 10 wherein the non-polar solvent is a hydrocarbon and the polar solvent is an alkanol.

12. The process of Claim 11 wherein the alkaline-earth metal oxide is calcium oxide, the alkaryl sulfonic acid is a benzene or naphthalene derivative wherein the alkyl groups contain 10 to 20 carbon atoms, the alkanol is isopropanol or isobutanol and the amount of water initially present in the reaction is about 0.15 to about 0.5 weight percent.

13. The process of Claim 12 wherein the sulfonic acid is an alkylbenzene sulfonic acid and the alkyl groups contain 10 to 14 carbon atoms, and the alkanol is isopropanol.

14. The process of Claim 13 wherein the alkyl benzene sulfonic acid is dodecylbenzene sulfonic acid.

15. The process of Claim 1 wherein the alkaline-earth metal oxide and alkaryl sulfonic acid are reacted together in the presence of an alkaline-earth metal alkaryl sulfonate solution containing less than 5 weight percent water, about 20 to 80 weight percent of alkaline-earth metal oxide alkaryl sulfonate, the remainder being solvent, in amounts to provide at least 0.1 weight percent water.

16. The process of Claim 15 wherein the alkyl groups of the alkaryl sulfonic acid contain 3 to 30 carbon atoms, the polar solvent is an alkanol and there is at least 5 weight percent of the solution containing 40 to 70 weight percent alkaline-earth metal alkaryl sulfonate and 0.1 to about 4.0 weight percent water, added to the reaction prior to addition of the alkaryl sulfonic acid that is proportioned into the mixture of the solvent, alkaline-earth metal oxide and alkaline-earth metal sulfonate.

17. The process of Claim 16 wherein the alkaline-earth metal oxide is calcium oxide, the alkaryl sulfonic acid is a benzene or naphthalene derivative wherein the alkyl groups contain 10 to 20 carbon atoms and the alkanol is isopropanol or isobutanol.

18. The process of Claim 17 wherein the sulfonic acid is an alkyl benzene sulfonic acid wherein the groups contain 10 to 14 carbon atoms, the alcohol is isopropanol, and the solution is a reaction product of Claim 1.

19. The process of Claim 18 wherein the alkyl benzene sulfonic acid is dodecylbenzene sulfonic acid and the solvent medium contains at least 15 weight percent isopropanol and a non-polar hydrocarbon solvent.

20. The process of Claim 11 wherein the non-polar solvent is diesel fuel oil ASTM #2.

**Patentansprüche**

1. Verfahren zur Herstellung von Erdalkalimetall-Alkaryl-Sulfonat-Lösungen mit 1,5 bis etwa 3,5 Gew.-% Wasser, wobei ein Erdalkalimetall-Oxyd mit einer Alkaryl-Sulfonsäure in einem Medium mit einem polaren Lösungsmittel in Gegenwart von mindestens 0,05 bis höchstens etwa 2,5 Gew.-% Wasser.

2. Verfahren nach Anspruch 1, dadurch gekennzeichnet, daß die Alkaryl-Sulfonsäuren Alkyl-Gruppen mit 3 bis 30 Kohlenstoffatomen aufweisen, das polare Lösungsmittel ein Alkanol ist und die verwendete Wassermenge etwa 0,1 bis 2 Gew.-% beträgt.

3. Verfahren nach Anspruch 2, dadurch gekennzeichnet, daß das Erdalkalimetalloxyd, die Alkaryl-Sulfonsäure ein Benzon oder Naphthalin-Derivat ist, wobei die Alkylgruppe 10 bis 20 Kohlenstoffatome enthält, und die das Alkanol Isopropanol oder Isobutanol ist und die Menge des anfänglich vorhandenen Wassers während der Reaktion etwa 0,15 bis 0,5 Gew.-% beträgt.

4. Verfahren nach Anspruch 3, dadurch gekennzeichnet, daß die Sulfonsäure eine Alkylbenzon-Sulfonsäure ist, wobei die Alkylgruppen 10 bis 14 Kohlenstoffatome enthalten und das Alkanol Isopropanol ist.

5. Verfahren nach Anspruch 4, wobei die Alkaryl-Benzon-Sulfonsäure Dodecylbenzol-Sulfonsäure ist.

6. Verfahren nach Anspruch 2, bei dem die Alkaryl-Sulfonsäure in einer Mischung aus dem Lösungsmedium, Erdalkalimetalloxyd und Wasser in einer Rate proportioniert ist, um wenigstens die Reaktion des Erdalkalimetalloxyds und der Sulfonsäure zur Bildung des Salzes aufrecht zu erhalten.

7. Verfahren nach Anspruch 6, wobei das Erdalkalimetalloxyd Kalziumoxyd, die Alkaryl-Sulfonsäure ein Benzol oder Naphthalin-Derivat ist, wobei die Alkylgruppen 10 bis 20 Kohlenstoffatome enthalten, und wobei das Alkanol Isopropanol oder Isobutanol und die während der Reaktion vorhandene Wassermenge größer als 0,15 Gew.-% ist.

8. Verfahren nach Anspruch 7, bei dem die Sulfonsäure eine Alkylbenzol-Sulfonsäure ist, wobei die Alkylgruppen 10 bis 14 Kohlenstoffatome enthalten, und der Alkohol Isopropanol ist.

9. Verfahren nach Anspruch 8, bei dem die Alkylbenzon-Sulfonsäure Dedocylbenzon-Sulfonsäure ist.

10. Verfahren nach Anspruch 6, bei dem das Lösungsmedium ein unpolares und ein polares organisches Lösungsmittel umfaßt, wobei das polare Lösungsmittel in einer Menge von wenigstens 15 Gew.-% im Lösungsmedium vorhanden ist.

11. Verfahren nach Anspruch 10, wobei das nicht polare Lösungsmittel ein Kohlenwasserstoff und das polare Lösungsmittel ein Alkanol ist.

12. Verfahren nach Anspruch 11, bei dem das Erdalkalimetalloxyd Kalziumoxyd, die Alkaryl-Sulfonsäure ein Benzol oder Naphthalin-Derivat, wobei die Alkylgruppen 10 bis 20 Kohlenstoffatome enthalten, und das Alkanol Isopropanol oder Isobutanol ist, und wobei die anfänglich in der Reaktions vorhandene Wassermenge etwa 0,15 bis etwa 0,5 Gew.-% beträgt.

13. Verfahren nach Anspruch 12, bei dem die Sulfonsäure eine Alkylbenzol-Sulfonsäure ist, und die Alkylgruppen 10 bis 14 Kohlenstoffatome enthalten, und wobei das Alkanol Isopropanol ist.

14. Verfahren nach Anspruch 13, bei dem die Alkylbenzol-Sulfonsäure Dedocylbenzol-Sulfonsäure ist.

15. Verfahren nach Anspruch 1, bei dem das Erdalkalimetalloxyd und die Alkaryl-Sulfonsäure miteinander in Anwesenheit einer Erdalkalimetall-Alkaryl-Sulfonlösung miteinander reagieren, wobei die Erdalkalimetall-Alkaryl-Sulfonlösung weniger als 5 Gew.-% Wasser, etwa 20 bis 80 Gew.-% Erdalkalimetalloxyd-Alkaryl-Sulfonat enthält und der Rest Lösungsmittel in einer Menge ist, um wenigstens 0,1 Gew.-% Wasser vorzusehen.

16. Verfahren nach Anspruch 15, bei dem die Alkylgruppen der Alkaryl-Sulfonsäure 3 bis 30 Kohlenstoffatome enthält, das polare Lösungsmittel ein Alkanol ist, und wenigstens 5 Gew.-% der Lösung, die 40 bis 70 Gew.-% Erdalkalimetall-Alkaryl-Sulfonat und 0,1 bis etwa 4,0 Gew.-% Wasser enthält, der Reaktion vor Zugeben der Alkaryl-Sulfonsäure zugesetzt wird, die in die Mischung des Lösungsmittels, des Erdalkalimetalloxyds und des Erdalkalimetallsulfonats proportioniert ist.

17. Verfahren nach Anspruch 16, bei dem das Erdalkalimetalloxyd Kalziumoxyd, die Alkaryl-Sulfonsäure ein Benzol oder Naphthalin-Derivat, wobei die Alkylgruppen 10 bis 20 Kohlenstoffatome enthalten, und der Alkanol Isopropanol oder Isobutanol ist.

18. Verfahren nach Anspruch 17, wobei die Sulfonsäure eine Benzolsulfonsäure mit 10 bis 14 Kohlenstoffatomen enthaltenen Gruppen, der Alkohol Isopropanol und die Lösung ein Reaktionsprodukt von Anspruch 1 ist.

19. Verfahren nach Anspruch 18, bei dem die Alkylbenzol-Sulfonsäure Dedocylbenzol-Sulfonsäure ist und das Lösungsmedium wenigstens 15 Gew.-% Isopropanol und ein unpolares Kohlenwasserstoff-Lösungsmittel enthält.

20. Verfahren nach Anspruch 11, wobei das unpolare Lösungsmittel Dieselöl ASTM Nr. 2 ist.

## Revendications

1. Procédé de préparation de solutions d'alkaryl sulfonate de métal alcalino-terreux, contenant de 1,5 à environ 3,5 pour cent en poids d'eau, ce procédé consistant à faire réagir ensemble un oxyde de métal alcalino-terreux avec un acide alkaryl sulfonique dans un milieu contenant un solvant polaire, en présence de plus de 0,05 à moins d'environ 2,5 pour cent en poids d'eau.

2. Procédé selon la revendication 1, dans lequel les acides alkaryl sulfoniques possèdent des groupes alkyle contenant de 3 à 30 atomes de carbone, le solvant polaire est un alcanol, et la quantité d'eau utilisée va d'environ 0,1 à moins d'environ 2 pour cent en poids.

3. Procédé selon la revendication 2, dans lequel l'oxyde de métal alcalino-terreux est l'oxyde de calcium, l'acide alkaryl sulfonique est un dérivé du benzène ou du naphtalène, dont les groupes alkyle contiennent de 10 à 20 atomes de carbone, l'alcanol est l'isopropanol ou l'isobutanol, et la quantité d'eau initialement présente pendant la réaction va d'environ 0,15 à 0,5 pour cent en poids.

4. Procédé selon la revendication 3, dans lequel l'acide sulfonique est un acide alkyl benzène sulfonique, dont les groupes alkyle contiennent de 10 à 14 atomes de carbone, et l'alcanol est l'isopropanol.

5. Procédé selon la revendication 4, dans lequel l'acide alkylbenzène sulfonique est l'acide dodécylbenzène sulfonique.

6. Procédé selon la revendication 2, dans lequel l'acide alkaryl sulfonique est ajusté dans un mélange dudit milieu solvant, d'oxyde de métal alcalino-terreux et d'eau, à un taux convenant pour au moins entretenir la réaction de l'oxyde de métal alcalino-terreux et de l'acide sulfonique pour former le sel de ce dernier.

7. Procédé selon la revendication 6, dans lequel l'oxyde de métal alcalino-terreux est l'oxyde de calcium, l'acide alkaryl sulfonique est un dérivé du benzène ou du naphtalène, dont les groupes alkyle contiennent de 10 à 20 atomes de carbone, l'alcanol est l'isopropanol ou l'isobutanol, et la quantité d'eau présente pendant la réaction est supérieure à 0,15 pour cent en poids.

8. Procédé selon la revendication 7, dans lequel l'acide sulfonique est un acide alkylbenzène sulfonique, dont les groupes alkyle contiennent de 10 à 14 atomes de carbone, et l'alcool est l'isopropanol.

9. Procédé selon la revendication 8, dans lequel l'acide alkylbenzène sulfonique est l'acide dodécylbenzène sulfonique.

10. Procédé selon la revendication 6, dans lequel le milieu solvant comprend un solvant organique non polaire et un solvant organique polaire, le solvant polaire étant présent dans le milieu solvant en une quantité d'au moins 15 pour cent en poids.

11. Procédé selon la revendication 10, dans lequel le solvant non polaire est un hydrocarbure et le solvant polaire est un alcanol.

12. Procédé selon la revendication 11, dans lequel l'oxyde de métal alcalino-terreux est l'oxyde de calcium, l'acide alkaryl sulfonique est un dérivé du benzène ou du naphtalène, dont les groupes alkyle contiennent de 10 à 20 atomes de carbone, l'alcanol est l'isopropanol ou l'isobutanol, et la quantité d'eau initialement présente dans le système réactionnel va d'environ 0,15 à environ 0,5 pour cent en poids.

13. Procédé selon la revendication 12, dans lequel l'acide sulfonique est un acide alkylbenzène sulfonique et les groupes alkyle contiennent de 10 à 14 atomes de carbone, et l'alcanol est l'isopropanol.

14. Procédé selon la revendication 13, dans lequel l'acide alkylbenzène sulfonique est l'acide dodécylbenzène sulfonique.

15. Procédé selon la revendication 1, dans lequel on fait réagir ensemble l'oxyde de métal alcalino-terreux et l'acide alkaryl sulfonique, en présence d'une solution d'alkaryl sulfonate de métal alcalino-terreux contenant moins de 5 pour cent en poids d'eau, environ 20 à 80 pour cent en poids d'alkaryl sulfonate de métal alcalino-terreux, le reste étant du solvant, en des quantités permettant d'avoir au moins 0,1 pour cent en poids d'eau.

16. Procédé selon la revendication 15, dans lequel les groupes alkyle de l'acide alkaryl sulfonique contiennent de 3 à 30 atomes de carbone, le solvant polaire est un alcanol et il y a au moins 5 pour cent en poids de la solution contenant 40 à 70 pour cent en poids d'alkaryl sulfonate de métal alcalino-terreux et 0,1 à environ 4,0 pour cent en poids d'eau, qui sont ajoutés au système réactionnel avant l'addition de l'acide alkaryl sulfonique qui est ajusté dans le mélange du solvant, de l'oxyde de métal alcalino-terreux et du sulfonate de métal alcalino-terreux.

17. Procédé selon la revendication 16, dans lequel l'oxyde de métal alcalino-terreux est l'oxyde de calcium, l'acide alkaryl sulfonique est un dérivé du benzène ou du naphtalène, dont les groupes alkyle contiennent de 10 à 20 atomes de carbone, et l'alcanol est l'isopropanol ou l'isobutanol.

18. Procédé selon la revendication 17, dans lequel l'acide sulfonique est un acide alkylbenzène sulfonique, dont les groupes alkyle contiennent de 10 à 14 atomes de carbone, l'alcool est l'isopropanol et la solution est un produit de réaction tel que défini à la revendication 1.

19. Procédé selon la revendication 18, dans lequel l'acide alkylbenzène sulfonique est l'acide dodécylbenzène sulfonique, et le milieu solvant contient au moins 15 pour cent en poids d'isopropanol et un solvant hydrocarboné non polaire.

20. Procédé selon la revendication 11, dans lequel le solvant non polaire est le carburant Diesel ASTM #2.